# EUROPEAN PATENT SPECIFICATION

(11) **EP 1 245 683 B1**
(45) Date of publication and mention of the grant of the patent: **14.06.2006**
(21) Application number: 01302991.3
(22) Date of filing: 29.03.2001
(51) Int. Cl.: C12Q 1/68

(54) **Method for making a recombinant S. cerevisiae by using essential and regulatory genes of mycobacteria**
Verfahren zur Herstellung einer rekombinanten Hefe S. cerevisiae durch die Verwendung von wesentlichen und regulatorischen Genen von Mykobakterien
Procédé pour la préparation d'un S. cerevisiae récombinant par l'utilisation de gènes des Mycobactérie essentiels et régulatoirs

(43) Date of publication of application: 02.10.2002
(73) Proprietor: COUNCIL OF SCIENTIFIC AND INDUSTRIAL RESEARCH, New Delhi 110 001 (IN)
(72) Inventor: Soni, Vishal, Chandigarh 160036 (IN); Khandrika, Lakshami Pathi, Chandigarh 160036 (IN); Agrawal, Pushpa, Chandigarh 160036 (IN)
(74) Representative: Gaunt, Robert John

(56) References cited:
- WO-A-94/28137
- DATABASE BIOSIS [Online] BIOSCIENCES INFORMATION SERVICE, PHILADELPHIA, PA, US; 1999 MULDER N J ET AL: "Characterization of a Mycobacterium tuberculosis homologue of the Streptomyces coelicolor whiB gene." Database accession no. PREV200000002199 XP002180394 & TUBERCLE AND LUNG DISEASE, vol. 79, no. 5, 1999, pages 299-308, ISSN: 0962-8479
- COLE S T ET AL: "Deciphering the biology of Mycobacterium tuberculosis from the complete genome sequence" NATURE, MACMILLAN JOURNALS LTD. LONDON, GB, vol. 393, no. 6685, 11 June 1998 (1998-06-11), pages 537-544, XP002087941 ISSN: 0028-0836
- GOMEZ J.E. ET AL.: "whmD is an essential mycobacterial gene required for proper septation and cell division" PROCEEDINGS OF THE NATIONAL ACADEMY OF SCIENCES, vol. 97, no. 15, 18 July 2001 (2001-07-18), pages 8554-8559, XP002180393 US
- DATABASE SWISS-PROT [Online] Accession Number: O53353, 1 June 1998 (1998-06-01) COLE S.T. ET AL.: "Deciphering the biology of Mycobacterium tuberculosis from the complete genome sequence" XP002180395
- SRIVASTAVA R ET AL: "Beta-galactosidase reporter system in mycobacteria and its application in rapid antimycobacterial drug screening;" BIOCHEM.BIOPHYS.RES.COMMUN.;(1997) 235, 3, 602-05 CODEN: BBRCA9 ISSN: 0006-291X, XP002180396 Cent.Drug-Res.Inst.Lucknow

### SEQUENCE LISTING

<110> Council of Industrial & Scientific Research
<120> A Reporter Gene Based Method for the Screening of Anti-Tuberculosis Drugs by Using Essential and Regulatory Genes of Mycobacteria as Drug Target
<130> RJG/2816-EP
<140> EP01302991.3
   <141> 2001-03-29
<160> 16
<170> PatentIn Ver. 2.1
<210> 1
   <211> 255
   <212> DNA
   <213> Artificial Sequence
<220>
   <223> Description of Artificial Sequence: oligonucleotide primer
<400> 1
   atggattggc gccacaaggc ggtctgtcgt gacgaggatc cggaactgtt cttcccggta 60
   ggaaacagtg gtccggcact tgcgcagatc gctgacgcga aactggtctg taatcggtgc 120
   ccggtcacca cagagtgcct cagctgggca ctgaataccg gccaggactc gggcgtctgg 180
   ggaggcatga gcgaagacga gcggcgcgcg ctgaagcgtc gcaacgcccg cacgaaagcc 240
   cgtaccgggg tctga                                                  255
<210> 2
   <211> 89
   <212> PRT
   <213> Mycobacterium tuberculosis
<400> 2
   Met Val Pro Glu Ala Pro Ala Pro Phe Glu Glu Pro Leu Pro Pro Glu
    1               5                   10                  15
   Ala Thr Asp Gln Trp Gln Asp Arg Ala Leu Cys Ala Gln Thr Asp Pro
               20                  25                  30
   Glu Ala Phe Phe Pro Glu Lys Gly Gly Ser Thr Arg Glu Ala Lys Lys
           35                  40                  45
   Ile Cys Met Gly Cys Glu Val Arg His Glu Cys Leu Glu Tyr Ala Leu
       50                  55                  60
   Ala His Asp Glu Arg Phe Gly Ile Trp Gly Gly Leu Ser Glu Arg Glu
   65                  70                  75                  80
   Arg Arg Arg Leu Lys Arg Gly Ile Ile
                   85
<210> 3
   <211> 270
   <212> DNA
   <213> Mycobacterium tuberculosis
<400> 3
   tcagatgatc ccgcgtttga ggcggcggcg ctcgcgttcg gaaagaccac cccagatgcc 60
   gaaccgctcg tcatgagcca gggcgtactc cagacactcg tgccgcacct cgcagcccat 120
   gcaaatcttc ttggcctcac gcgtggagcc gcccttctcc gggaagaacg cttcgggatc 180
   cgtttgcgca catagcgcac ggtcctgcca ttggtcggtg gcttccggcg gcagaggttc 240
   ctcgaatggc gccggcgcct cgggaaccaa                                  270
<210> 4
   <211> 102
   <212> PRT
   <213> Mycobacterium tuberculosis
<400> 4
   Met Pro Gln Pro Glu Gln Leu Pro Gly Pro Asn Ala Asp Ile Trp Asn
   1               5                   10                  15
   Trp Gln Leu Gln Gly Leu Cys Arg Gly Met Asp Ser Ser Met Phe Phe
               20                  25                  30
   His Pro Asp Gly Glu Arg Gly Arg Ala Arg Thr Gln Arg Glu Gln Arg
           35                  40                  45
   Ala Lys Glu Met Cys Arg Arg Cys Pro Val Ile Glu Ala Cys Arg Ser
       50                  55                  60
   His Ala Leu Glu Val Gly Glu Pro Tyr Gly Val Trp Gly Gly Leu Ser
   65                  70                  75                  80
   Glu Ser Glu Arg Asp Leu Leu Leu Lys Gly Thr Met Gly Arg Thr Arg
                   85                  90                  95
   Gly Ile Arg Arg Thr Ala
               100
<210> 5
   <211> 309
   <212> DNA
   <213> Mycobacterium tuberculosis
<400> 5
   atgccacagc cggagcagct accgggaccc aacgcagaca tctggaactg gcaattgcaa 60
   ggcctgtgtc gcggcatgga ctcatcgatg ttcttccatc ccgacggcga gcgtggccgt 120
   gcccgaacgc agcgcgaaca acgcgccaag gaaatgtgtc ggcgctgccc cgtgatcgag 180
   gcgtgccgat cccatgcgtt agaggtcggt gagccctatg gcgtttgggg tggcctgtcc 240
   gaatccgagc gcgacctact cctcaagggc accatgggac gcacccgcgg catccgccgc 300
   acagcttaa                                                         309
<210> 6
   <211> 100
   <212> PRT
   <213> Mycobacterium tuberculosis
<400> 6
   Met Val Arg Ser Val Asp Ala Glu Glu Arg Ile Ala Trp Val Ser Lys
   1               5                   10                  15
   Ala Leu Cys Arg Thr Thr Asp Pro Asp Glu Leu Phe Val Arg Gly Ala
               20                  25                  30
   Ala Gln Arg Lys Ala Ala Val Ile Cys Arg His Cys Pro Val Met Gln
           35                  40                  45
   Glu Cys Ala Ala Asp Ala Leu Asp Asn Lys Val Glu Phe Gly Val Trp
       50                  55                  60
   Gly Gly Met Thr Glu Arg Gln Arg Arg Ala Leu Leu Lys Gln His Pro
    65                  70                  75                  80
   Glu Val Val Ser Trp Ser Asp Tyr Leu Glu Lys Arg Lys Arg Arg Thr
                    85                  90                  95
   Gly Thr Ala Gly
               100
<210> 7
   <211> 303
   <212> DNA
   <213> Mycobacterium tuberculosis
<400> 7
   ctatccggcg gtgccggtgc ggcgcttgcg cttctcaagg tagtccgacc acgaaaccac 60
   ctcgggatgt tgcttgagca gagccctgcg ctggcgctcg gtcatgccac cccaaacacc 120
   gaactcgacc ttgttgtcca gcgcatctgc cgcacactct tgcattaccg gacagtgacg 180
   gcagatcacc gcggccttgc gttgtgcggc tcctcgaaca aagagttcgt cagggtcggt 240
   agtccggcac agcgccttgg atacccacgc gatccgctct tccgcgtcta cgctgcgtac 300
   cac                                                               303
<210> 8
   <211> 20
   <212> DNA
   <213> Artificial Sequence
<220>
   <223> Description of Artificial Sequence: oligonucleotide primer
<400> 8
   acccgttacc agccaagaag 20
<210> 9
   <211> 19
   <212> DNA
   <213> Artificial Sequence
<220>
   <223> Description of Artificial Sequence: oligonucleotide primer
<400> 9
   gggacggttg atgctgtag 19
<210> 10
   <211> 17
   <212> DNA
   <213> Artificial Sequence
<220>
   <223> Description of Artificial Sequence: oligonucleotide primer
<400> 10
   ggccgggtca gatgatc 17
<210> 11
   <211> 17
   <212> DNA
   <213> Artificial Sequence
<220>
   <223> Description of Artificial Sequence: oligonucleotide primer
<400> 11
   accgcatctg agtttgg 17
<210> 12
   <211> 22
   <212> DNA
   <213> Artificial Sequence
<220>
   <223> Description of Artificial Sequence: oligonucleotide primer
<400> 12
   atgccacagc cggagcagct ac 22
<210> 13
   <211> 21
   <212> DNA
   <213> Artificial Sequence
<220>
   <223> Description of Artificial Sequence: oligonucleotide primer
<400> 13
   ttaagctgtg cggcggatgc c 21
<210> 14
   <211> 21
   <212> DNA
   <213> Artificial Sequence
<220>
   <223> Description of Artificial Sequence: oligonucleotide primer
<400> 14
   ctatccggcg gtgccggtgc g 21
<210> 15
   <211> 21
   <212> DNA
   <213> Artificial Sequence
<220>
   <223> Description of Artificial Sequence: oligonucleotide primer
<400> 15
   gtggtacgca gcgtagacgc g 21
<210> 16
   <211> 84
   <212> PRT
   <213> Mycobacterium tuberculosis
<400> 16
   Met Asp Trp Arg His Lys Ala Val Cys Arg Asp Glu Asp Pro Glu Leu
    1               5                  10                  15
   Phe Phe Pro Val Gly Asn Ser Gly Pro Ala Leu Ala Gln Ile Ala Asp
               20                  25                  30
   Ala Lys Leu Val Cys Asn Arg Cys Pro Val Thr Thr Glu Cys Leu Ser
           35                  40                  45
   Trp Ala Leu Asn Thr Gly Gln Asp Ser Gly Val Trp Gly Gly Met Ser
       50                  55                  60
   Glu Asp Glu Arg Arg Ala Leu Lys Arg Arg Asn Ala Arg Thr Lys Ala
    65                 70                  75                  80
   Arg Thr Gly Val

## Description

### FIELD OF THE INVENTION

This invention relates to a method for making a recombinant *S. cerevisiae*. It can be used in a reporter gene based drug screening system against tuberculosis by using essential regulatory genes of *Mycobacterium tuberculosis* H37Rv as the target. Such use is not part of the invention. Such regulatory genes are the *whiB* genes of mycobacteria whose functions are essential for the survival and normal growth of mycobacteria.

### BACKGROUND OF THE INVENTION

The Streptomycetes are dimorphic organisms. After reaching the late log phase of growth, the substrate mycelia differentiate into the aerial mycelia. The tip of the aerial mycelium then differentiates into a chain of spores. Each spore represents a single cell and is separated by a septum. In 1992, Davis and Chater (Davis, N.K. and Chater, K.F. 1992. Mol. Gen. Genet. 232: 352-358) reported that any mutation in the *whiB* gene of *Streptomyces coelicolor* A3(2) results in a non the sporulating organism. These mutants were also white in colour since they had lost the capability to produce deep reddish blue pigment which is a characteristic of the wild type strain of *Streptomyces coelicolor* A3(2). It was further confirmed that a fully functional *whiB* gene is essential for the sporulation of the *Streptomyces coelicolor* A3(2) and *whiB* gene may be a transcription activator. A *whiB* homologue was also reported from *Streptoverticillium sps, Streptomyces aurofaciens* and *Rhodococcus opacus* ( Kormanec and Homerova, 1993 Nucl. Acid Res. 21:2512; Seibert,V., Kourbatova, E.M., Golovvela, L.M. and M. Schlomann. 1998. 180: 3503-3508; Soliveri, J.E., Vijgenboom, E., Granozzi, C., Plaskitt, K.A. and K.F. Chater. 1993. J.Gen.Microbiol. 139:2569-2578). However, unexpectedly, the genome sequence of *Mycobacterium tuberculosis* H37Rv showed the presence of four genes that is *whiB1*/Rv3219-254bp, *whiB2*/Rv3260c-269bp, *whiB3*/Rv 3416-308bp and *whiB4*/Rv3681c-302bp whose deduced amino acid products were similar to the *whiB* gene of *Streptomyces coelicolor* A3(2). The amino acid sequences of *whiB* genes of *M. tuberculosis* H37Rv show 32-35 per cent homology to the amino acid sequences of *Streptomyces whiB* genes. Although the homology is relatively low, the general property of the predicted protein remains conserved. General morphology of mycobacteria is bacillus unlike the species of streptomycetes, which are filamentous. So far, sporulation of mycobacteria has not been reported. Therefore the presence of *whiB* like genes, which control the sporulation, in a non-sporulating organism is highly intriguing. The predicted amino acid sequence of *whiB* gene suggests that the *whiB* gene may code for a transcription activator. If that were so then *whiB* genes would be a regulatory gene. However so far it has not been reported that the *whiB* genes indeed code for a transcription activator. If the *whiB* genes are indeed a set of regulatory genes then the question is what kind of genes do they control? Recently, Gomez and Bishai (Gomez, J.E. and Bishai, W.R., 2000. Proc. Natl. Acad. Sci. 97: 8554-8559) have shown that a *whiB2* homologue of *Mycobacterium tuberculosis* H37Rv is present in *Mycobacterium smegmatis* and is essential for its survival. *Mycobacterium smegmatis* is a fast growing and non-pathogenic organism. However no report or patent could be found related to the invention described in this application.

The present patent describes that out of the four *whiB* genes originally described in the *Mycobacterium tuberculosis* H37Rv genome sequence, the *whiB1* /Rv 3219 is essential to the survival of the *Mycobacterium bovis* BCG and *whiB3* /Rv 3416 appears to control septa formation during cell division. The properties of these genes were not reported in the genome sequence of *Mycobacterium tuberculosis* H37Rv nor have they been published in the literature (Cole et al. Nature 1998. 393: 537-544.).

Tuberculosis is still one of the major killers of human lives in India and in most of the developing countries. The spread of HIV has compounded the problems of tuberculosis because several species of mycobacteria, which were otherwise not known to infect humans, have also been found to be associated with the HIV infected patients. More than often these new pathogens are not sensitive to the conventional therapy of tuberculosis. Further the incidence of the tuberculosis caused by the drug resistant mycobacteria is also increasing at an alarming rate. These resistant bacteria do not respond to conventional therapy. Thus there is a need to find either new drugs or first to find new drug targets that have not yet developed the capability to modify themselves and then search for a drug, which would attack at a particular target.

The search for a new drug without any specific target usually leads to the rediscovery of known drugs. Secondly, the mode of action of the new drug discovered by random search is usually not known, thus the study of pharmaco-kinetics can be a laborious process. Further, if the organism develops resistance to the new drug as well then modification of these drugs which would suit to the target of resistant organism would be very difficult. With the advancement in computer simulation studies and modeling software, it is possible to design a drug if the target is known.

The present invention relates to a method for making recombinant S. cerevisiae. The DNA binding property of the *whiB* genes of *Mycobacterium tuberculosis* H37Rv that has been demonstrated for the first time in this invention. The method herein described is based on the activation of the reporter gene *lacZ,* which produces the enzyme β-galactosidase. In a normal circumstance the β-galactosidase activity remain repressed because the *whiB* genes, which code for a DNA binding protein, can bind to the *lexA* operators present in certain strains of *Saccharomyces cerevisiae* as well as in reporter plasmids. Binding of *whiB* gene products with the operator's sequence results in the repression of the transcription of the *lacZ* gene, which produces β-galactosidase enzyme. However if a drug binds to the *whiB* genes then its products will not be available for binding to the operators and the *lacZ* transcription will continue. Activation or repression of β-galactosidase can be monitored either by adding 5-Bromo-4-chloro-3-indolyl β-D-galactopyranoside (X-gal) into the growth medium, which produces blue color or by adding o-nitrophenol β-D-galactopyranoside (ONPG) which produces yellow color in presence of β-galactosidase. Intensity of the color production is directly related to the activation of β-galactosidase enzyme and therefore would be directly related to the binding capability of a drug. In other words, a strong binder to the target will allow strong activation of β-galactosidase, however, a poor binder to the target would permit low activation of β-galactosidase enzyme. The described method is fully compatible to any automated High-Through-Put screening system or any other automated or non-automated screening system.

The present invention is thus based upon the need to find a new drug target in *Mycobacterium tuberculosis* H37Rv and develop a drug screening system based upon the identified target. To suit the fast developing technology and urgent need to find a better cure, it is desired that the screening system herein described compatible to any automated High-Through-Put screening or any other mechanised screening procedure.

### OBJECTS OF THE INVENTION

The objective of the present invention is to provide a method for making a recombinant S. cerevisiae, which would facilitate target specific screening of anti-tuberculosis drugs.

Also described is the development of a drug screening system, which is fast and uses the properties of the target gene.

### SUMMARY OF THE INVENTION

The present invention relates to a method for making a recombinant S.cerevisiae by transformation with the whiB genes of Mycobacteria having the sequences SEQ ID NO:1, 3, 5 and 7. Also described is a drug screening method taking advantage of the yeast two-hybrid system, known in the literature. The patent also describes that the *whiB* genes are essential regulatory genes of *Mycobacterium tuberculosis* H37Rv and appear to be conserved amongst the pathogenic and slow growing mycobacteria. Thus the novelty of the described method is to use the *whiB* genes which are regulatory genes of mycobacteria, whose functions have not been reported so far, as drug target by using the yeast *Saccharomyces cerevisiae* as a surrogate host.

### DETAILED DESCRIPTION OF THE INVENTION

To accomplish the aforesaid objective, it is essential to demonstrate that a functional target gene is essential for the survival of the *Mycobacterium tuberculosis* H37Rv. The sporulation gene homologue of *Streptomyces coelicolor* A3(2) was found to be present in *Mycobacterium tuberculosis* H37Rv. Since mycobacteria are not known to sporulate, it was assumed that these sporulation homologues would have yet unknown but important function. It has been assumed that the *whiB* genes may act as a transcription activator and therefore would have regulatory function. It is further assumed that a regulatory gene, which controls sporulation, yet is present in a non-sporulating organism, may indeed be an essential gene.

The drug screening method described in this patent takes advantage of the yeast two-hybrid system, known in the literature. The patent also describes that the *whiB* genes are essential regulatory genes of *Mycobacterium tuberculosis* H37Rv and appear to be conserved amongst the pathogenic and slow growing mycobacteria. Thus the novelty of the described method is to use the *whiB* genes which are regulatory genes of mycobacteria, whose function have not been reported so far, as drug target by using the yeast *Saccharomyces cerevisiae* as a surrogate host.

*Mycobacterium tuberculosis* H37Rv is a slow growing organism and is highly virulent. Thus, the use of live organism poses a severe health hazard. Therefore it was essential to develop a screening system in a different host organism, which is non-pathogenic, fast growing and also to provide the method that is adaptable to High-Through-Put screening or any other automated screening systems.

Accordingly, a reporter gene based method is described for the screening of anti-tuberculosis drugs comprising using *whiB* like genes *(whiB*1 *, whiB*2 whi*B*3 *whiB*4) present in *Mycobacterium tuberculosis* H37Rv, *Mycobacterium bovis* BCG and *Mycobactereium leprae* having DNA and protein sequence as shown in sequence ID 1 to 4 as drug targets for the screening of anti-tuberculosis and anti-leprosis drugs.

The *whiB*3 gene of *Mycobacterium tuberculosis* H37Rv and *Mycobacterium bovis* BCG can be used for the screening of anti-tuberculosis drugs. The presence of functional *whiB*4 gene of *Mycobacterium tuberculosis* H37Rv and *Mycobacterium bovis* BCG is important for their normal growth. The *whiB*4 gene of *Mycobacterium tuberculosis* H37Rv and *Mycobcaterium bovis* BCG code for a DNA binding protein.The drugs against *whiB*2 and the *whiB*4 genes of *Mycobacterium tuberculosis* H37Rv and *Mycobacterium bovis* BCG will be particularly useful where drug resistance has developed against the *whiB*1 and *whiB*3 genes or where the *anti-whiB*1 and *anti-whiB*3 drugs are allergic or toxic. The source of *whiB* like genes can be *Mycobacterium avium, Mycobacterium fortuitum, Mycobacterium gastri, Mycobacterium kansasii, Mycobacterium leprae, Mycobacterium marinum, Mycobacterium microti, Mycobacterium phlei, Mycobacterium scrofulaceum, Mycobacterium smegmatis* and *Mycobacterium xenopi* and related organisms.

The present invention relates to a method for making a recombinant *Saccharomyces cerevisiae,* said method comprising the steps of:
a) amplifying the whiB-like genes of *Mycobacteria* by the polymerase chain reaction (PCR), wherein the whiB-like genes are selected from the group consisting of whiB1 (SEQ ID NO:1), whiB2 (SEQ ID NO:3), whiB3 (SEQ ID NO:5), and whiB4 (SEQ ID NO:7) and the Mycobacteria are selected from the group consisting of *Mycobacterium tuberculosis, Mycobacterium bovis, Mycobacterium avium, Mycobacterium fortuitum, Mycobacterium gastri, Mycobacterium kansasii, Mycobacterium leprae, Mycobacterium marinum, Mycobacterium microti, Mycobacterium phlei, Mycobacterium scrofulaceum, Mycobacterium smegmatis and Mycobacterium xenopi,* and wherein the whiB genes are amplified with primers carrying either EcoRl or BamHI restriction enzyme sites at the 5' end and a Xhol restriction enzyme site at the 3' end;
b) digesting the fragment as obtained in step a) with suitable restriction enzymes and then cloning the fragment into an *E. coli*/*Saccharomyces cerevisiae* shuttle vector pEG202 to produce the cloned gene as a *LexA* fusion protein and transforming the clone into E.coli; and
c) amplifying the clone and then transforming the recombinant *whiB1, whiB2, whiB3* and *whiB4*/*pEG202* into a strain of *Saccharomyces cerevisiae* which has *LexA* operators.

Further, described is a reporter gene based method for the screening of anti-tuberculosis drugs by using essential and regulatory genes of mycobacteria as the drug target and comprises the following steps:
(a) amplifying the *whiB* genes of *Mycobacterium tuberculosis* H37Rv by polymerase chain reaction (PCR);
(b) cloning the products obtained in step (a) into plasmid vectors such as pUC19 or pBluescript SK⁺ or SK⁻ and transforming into *E.coli;*
(c) amplifying *whiB* genes of *Mycobacterium bovis* BCG by using the oligonucleotide primers of *Mycobacterium tuberculosis* H37Rv;
(d) cloning the product obtained in step (c) into a plasmid vector pUC19 or pBluescript SK⁺ or SK⁻; and sequencing the products to confirm the sequence of cloned fragment;
(e) constructing a gene disruption integrative vector by inserting a kanamycin cassette in between the *whiB* gene sequences of *Mycobacterium tuberculosis* H37Rv;
(fj constructing a gene disruption integrative vector by inserting a kanamycin cassette in between the *whiB* gene sequences of *Mycobacterium bovis* BCG;
(g) amplifying *whiB* genes of *Mycobacterium tuberculosis* H37Rv by using oligonucleotide primers carrying either *Eco*RI, or *Bam*HI restriction enzyme sites at the 5' end and *Xho*I restriction enzyme site at the 3' end;
(h) digesting the fragment as obtained in step (g) with suitable restriction enzymes and then cloning the fragment into a *E. coli*/*Saccharomyces cerevisiae* shuttle vector pEG202 to produce the cloned gene as a *LexA* fusion protein and transforming the recombinant plasmid into *E.coli;*
(i) preparing large amount of the desired recombinant plasmid and then transforming the recombinant *whiB*1*, whiB*2*, whiB*3*,* and *whiB*4/pEG202 into the *Saccharomyces cerevisiae* strains EGY48 and EGY191,
(j) co-transforming the pJK101, pSH 18-34, pSH17-4, pJG4-5 and pRFHM vectors into the *Saccharomyces cerevisiae* strains EGY48 and EGY191 already harboring *whiB* genes in the pEG202 vector and then selecting for the clones, which complements for uracil and histidine + uracil or histidine + uracil + tryptophan auxotrophy;
(k) growing the *Saccharomyces cerevisiae* transformants in the yeast nitrogen base medium containing: galactose / raffinose+, ura-, BU salt+, X-gal+; his-, ura-. BU salt+, X-gal+, glucose; galactose / raffinose+, ura-, his-, leu-; glucose+, ura-, his-, leu-; galactose / raffinose+, ura-, his-, trp-, leu- and glucose+, ura-, his-, trp-. leu-plates, and
(l) using the DNA binding property of the *whiB*1*, whiB*2 and *whiB*3 for the screening of anti-tuberculosis drugs.

In an embodiment, the restriction enzymes are selected from *Eco*Rl, *Xma*I *Bam*HI*, Sal*I*, Nco*I*, Not*I*, Xho*I*, Sal*I and *Pst*I.

The *whiB*1gene is essential for the survival of *Mycobacterium tuberculosis* H37Rv and *Mycobacterium bovis* BCG.

The *winB*2 gene is essential for the normal growth of *Mycobacterium tuberculosis* H37Rv and *Mycobacterium bovis* BCG.

The *whiB3* gene is essential for the cell division of a *Mycobacterium tuberculosis* H37Rv and *Mvcobacterium bovis* BCG.

The *whiB4* gene is essential for the normal growth of *Mycobacterium tuberculosis* H37Rv and *Mycobacterium bovis* BCG.

The *whiB4* gene of *Mycobacterium tuberculosis* H37Rv and *Mycrobacterium bovis* BCG is a transcription activator.

The *whiB1, whiB2* and *whiB3* genes of *Mycobacterium tuberculosis* H37Rv and *Mycobacterium bovis* BCG code for a DNA binding protein.

The whiB1, whiB2 and whiB3 genes of *Mycobacterium tuberculosis* H37Rv and *Mycobacterium bovis* BCG repress the activation of β-galactosidase genes after binding to the Lex A operator or any other like operators.

The DNA binding domain of the *whiB* genes is situated within the 15 amino acids at the carboxy- terminus of the protein.

The DNA binding property of the *whiB* genes has been used to screen those anti- tuberculosis drugs which attack the *whiB* genes. The described method can be used for any gene, which codes for a DNA binding protein. The method is compatible to any High - Through- Put or automated screening system.

The *Mycobacterium tuberculosis* H37Rv genome sequence was obtained from the internet site http://www.sanger.ac.uk and the sequence of the *whiB* genes were recovered. The authors, (Cole et al. Nature, 1998:393, 537-543), have annotated these genes as: *whiB1*/Rv3219 (255bp), *whiB2*/Rv3260c (270bp), *whiB3*/Rv3416 *(*309bp) and *whiB4*/Rv3681c (303bp). The sequence of oligonucleotide primers designed to amplify the *whiB* genes were as follow:
F 5' acccgttaccagccaagaag 3' and R 5' gggacggttgatgctgtag 3' for *whiB*1
F5' ggccgggtcagatgatc 3' and R 5'accgcatctgagtttgg 3' for *whiB*2
F 5' atgccacagccggagcagctac 3' and R 5' ttaagctgtgcggcggatgcc 3' for *whiB*3
F 5' ctatccggcggtgccggtgcg 3' and R 5' gtggtacgcagcgtagacgcg 3' for *whiB*4
The sequences of the genes are shown as sequences ID 1 to 4 separately.

The PCR was done by standard procedure. The PCR products were separated on 1 per cent agarose gel and the amplified bands were removed by cutting the gel. The PCR amplified DNA was purified by using a commercially available purification kit. Cloning and then transformation of the PCR fragments were done by standard procedure. The sequence of cloned fragments was confirmed by standard procedure.

Once the identity of the cloned fragments was confirmed, the *Mycobacterium tuberculosis* H37Rv *whiB* genes were used as probe to check whether similar genes were present in the *Mycobacterium bovis* BCG or not. The *Mycobacterium bovis* BCG chromosomal DNA was prepared by a published method (G.P.S. Raghava, R.J.Solanki, V. Soni and P. Agrawal. Biotechniques. 2000. 29:108-116). A standard Southern hybridization protocol was followed to confirm the presence of *whiB* genes in *Mycobacterium bovis* BCG as well.

As described for the *Mycobacterium tuberculosis* H37Rv, all four *whiB* genes in *Mycobacterium bovis* BCG were also PCR amplified. The oligonucleotide primers and PCR conditions were identical in both the cases. The PCR products were separated in an agarose gel, purified, cloned and sequenced as described in case of *Mycobacterium tuberculosis* H37Rv.

The sequence alignment using commercial computer software confirmed that the *whiB* gene sequences of *Mycobacterium tuberculosis* H37Rv and *Mycobacterium bovis* BCG are identical.

By using commercially available computer software Gene-Runner, restriction enzymes sites within the *whiB* sequences were found. Based on the sequence analysis results following enzyme sites were selected to generate vectors that can be used for gene disruption in *Mycobacterium bovis* BCG by the process of homologous recombination.
To generate a vector, which could be used for gene disruption the following constructs were created:
(a) for the *whiB*1*,* kanamycin cassette was inserted at *Pvu*II site;
(b) for *whiB*2*,* two independent constructs were made at *Mlu*I and at *Hae*III sites;
(c) for *whiB*3 also two constructs were made at *Eco*RI site and at the *Cla*I site;
(d) for *whiB*4*,* at the *Sac*II site.

The *whiB* recombinant clones were digested with restriction enzymes. However, whenever the recombinant clone had more than one site for a particular enzyme then the purified *whiB* fragments were digested and then ligated with the kanamycin cassette, which codes for kanamycin resistance. The *E. coli* strain was transformed and clones were selected for ampicillin and kanamycin resistance. Since these clones do not have mycobacterial origin of replication they will not survive within the mycobacteria unless they are integrated in the mycobacteria genome. Electro-competent *Mycobacterium bovis* BCG cells were prepared using the art known in the literature and transformed with 1µg of purified vector DNA. In each tube 1ml of 7H9 Middlebrook's medium was added and incubated at 37°C for 48hrs. The culture was then plated on a 7H10 Middlebrook's medium containing both ampicillin and kanamycin and incubated at 37°C. After three weeks of growth the colonies were again plated on kanamycin plates and allowed to grow at 37°C. In the kanamycin plates the colonies could be seen only after 6 weeks of incubation which suggested that the *whiB* disruption is deleterious for the growth of *Mycobacterium bovis* BCG. Disruption of individual *whiB* genes had the following effects on the growth and survival of *Mycobacterium bovis* BCG:
1. the *whiB*1 disruption is lethal.
2. the *whiB*2 disruption makes the cells very slow growing and the colonies are very small in size.
3. the *whiB*3 disruption makes the cells mycelial which clearly suggests that this disruption is controlling septa formation.
4. the *whiB*4 disruption also had a similar effect like *whiB*2 disruption.

To demonstrate the nature of the *whiB* gene, the weast two-hybrid system was used. This art is well known in the literature. In principle the system has been developed in such way that the trans-activation domain and the DNA binding domains are in two different plasmids. Unless both the domains come together, protein-protein interaction will not take place and thus a gene will not get activated. However, the system also allows one to check whether the gene in question codes for DNA binding protein or a transcription activator.

The *whiB*1*, whiB*2 and *whiB*4 genes were PCR amplified using oligonucleotide primers, which had *Eco*RI site at their 5' end and *Xho*I site at their 3' end. The *whiB*3 gene was amplified with the primers having *Bam*HI site at the 5' end and *Xho*I site at the 3' end. After digesting with the appropriate restriction enzymes these genes were cloned into the *E.coli* / *Saccharomyces cerevisiae* shuttle vector pEG202. After selecting for ampicillin resistant colonies, the recombinant clones were selected for his colonies in *Saccharomyces cerevisiae* EGY 48 and EGY 191. The his- clones were then transformed with the plasmids: pJk101, pSH18-34, pJG4-5, and pRFHM either singly or in combinations. The clones which complemented either for uracil, uracil + histidine or uracil + histidine + tryptophan were selected. These clones were then tested for either activation or repression of β-galactosidase activity by growing them in the plates containing yeast-nitrogen based medium and following supplements:
(1) glucose ⁺, ura⁻ BU salt⁺ +X-gal ( no color)
(2) galactose/raffinose⁺, ura⁻ + BU salt⁺ + X-gal (no color by all the four *whiB* genes only when pJK101 is present but pJK101 produced bright blue color, suggesting that *whiB* genes are expressed in the *Saccharomyces cerevisiae* and repress the activation of *lacZ* gene.)
(3) glucose⁺, ura⁻,his⁻,leu⁻ (no growth)
(4) galactose / raffinose⁺, ura⁻, his, leu- (no growth of *whiB*1*, whiB*2*,* and *whiB*3 but some growth was seen *of whiB*4 in presence of pSH18-34)
(5) galactose/ raffinose+, ura-, his-, tryp-, leu- ( only *whiB*4 showed some growth after three days of incubation in presence of pSH18-34 and pJG4-5, suggesting that the *whiB*4 gene is a weak transcription activator)
(6) glucose+, ura-, his-, tryp-, leu- (no growth).

The method of the present invention is illustrated in the examples given below which should not, however, be construed to limit the scope of the present invention.

### Example 1

The *Mycobacterium tuberculosis* H37Rv chromosomal DNA was prepared as following a published method (G.P.S. Raghava, R.J.Solanki, V.Soni and P. Agrawal. Biotechniques. 2000. 29: 108-116).

All four *whiB* genes of *Mycobacterium tuberculosis* H37Rv were PCR amplified using a standard protocol. The oligonucleotide primers and PCR conditions were as follows:
F 5' acccgttaccagccaagaag 3' and R 5' gggacggttgatgctgtag 3' for *whiB*1
F5' ggccgggtcagatgatc 3' and R 5'accgcatctgagtttgg 3' for *whiB*2
F 5' atgccacagccggagcagctac 3' and R 5' ttaagctgtgcggcggatgcc 3' for *whiB*3
F 5' ctatccggcggtgccggtgcg 3' and R 5' gtggtacgcagcgtagacgcg 3' for *whiB*4

The PCR products were separated on 1 per cent agarose gel and the amplified bands were removed by cutting the gel. The PCR amplified DNA was purified by using a commercially available kit.

The ends of the purified DNA were phosphorylated using T4 Pol-Kinase and then cloned into the *Sma*I site of the dephosphorylated pUC19 vector. The recombinant plasmids were transformed into *E.coli* strain and selected for the colonies, which did not produce blue color. The white colonies were recovered from the plates and were grown on a fresh medium containing 100ug/ml ampicillin and 25ug/ml of kanamycin as a selection marker. The clones were sequenced using a standard method, well known in the art. Thus this example establishes the cloning and confirmation of the four *whiB* genes of *Mycobacterium tuberculosis* H37Rv.

### Example 2

The *Mycobacterium tuberculosis* H37Rv is a highly virulent strain. Therefore, in order to find a suitable working system a non-virulent but vaccine strain of *Mycobacterium bovis* BCG was checked for the presence of *whiB* homologues. Genomic DNA of *Mycobacterium bovis* BCG was digested with *Sac*I and *Bam*HI restriction enzymes and the DNA fragments were separated on an 1 per cent agarose gel. The DNA was transferred onto a Hybond Nylon membrane using the published protocol and then the DNA was fixed in the membrane at 80°C for 30minutes. The cloned *whiB* fragments from pUC19 were removed by *Xbal* and *Kpn*I digestion and the DNA was purified using an Gel Extraction kit. The purified fragments were then used for 32p dCTP labeling by random priming kit, which is commercially available. By using standard protocol of Southern hybridization it was confirmed that the four *whiB* genes homologues of *Mycobacterium tuberculosis* H37Rv are present in *Mycobacterium bovis* BCG. The four *whiB* genes of *Mycobacterium bovis* BCG were PCR amplified using oligonucleotide primers of *Mycobacterium tuberculosis* H37Rv. The PCR fragment were cloned and transformed into a *E.coli* strain as described for *Mycobacterium tuberculosis* H37Rv. The DNA sequence of the cloned fragment and sequence alignment using commercial computer software confirmed that the *whiB* gene sequence of *Mycobacterium tuberculosis* H37Rv and *Mycobacterium bovis* BCG is identical.

### Example 3

In order to demonstrate the function of *whiB* genes in the life cycle of mycobacteria, the four-*whiB* homologues were disrupted in *Mycobacterium bovis* BCG. By using commercially available computer software Gene-Runner, restriction enzymes sites within the *whiB* sequences were found. Based on the sequence analysis results, following restriction enzyme sites were selected to generate vectors that then can be used for gene disruption in *Mycobacterium bovis* BCG by the process of homologous recombination. To generate a vector, which could be used for gene disruption the following constructs were created:
(a) for the *whiB*1*,* kanamycin cassette was inserted at *Pvu*II site at the position 143;
(b) for *whiB*2*,* two independent constructs were made at *Mlu*I at the position 138 and at *Hae*III at the position 133;
(c) for *whiB*3, also two constructs were made at the *Eco*RI site at the position 52, and at the *Cla*I site at the position 85;
(d) for *whiB*4, insertion was at the *Sac*II site at position 192.

The *whiB* recombinant clones were digested with the restriction enzymes however whenever the recombinant clone had more than one site for a particular enzyme then the purified *whiB* fragments were digested and then ligated with the kanamycin cassette, which codes for kanamycin resistance. The *E. coli* strain was transformed and clones were selected for ampicillin and kanamycin resistance. Since these clones do not have mycobacterial origin of replication they will not survive within the mycobacteria unless they are integrated in the mycobacteria genome. Electro-competent *Mycobacterium bovis* BCG cells were prepared using the art known in the literature and transformed with 1µg of purified vector DNA. In each tube, 1ml of 7H9 Middlebrook's medium was added and incubated at 37°C for 48hrs. The culture was then plated on a 7H10 Middlebrook's medium containing both ampicillin and kanamycin and incubated at 37°C. After three weeks of growth, the colonies were again plated on only kanamycin plates and allowed to grow at 37°C. In the kanamycin plates, the colonies could be seen only after 6 weeks of incubation which suggested that the *whiB* disruption is deleterious for the growth of *Mycobacterium bovis* BCG. Disruption of individual *whiB* genes had the same effects on the growth and survival of *Mycobacterium bovis* BCG.

### Example 4

To demonstrate the nature of the *whiB* gene the yeast two-hybrid system was used. This art is well known in the literature. In principle the system has been developed where trans-activation domain and the DNA binding domains are in two different plasmids. Unless they come together protein-protein interaction will not take place thus a gene will not get activated. However, the system also allows one to check whether the gene in question codes for DNA binding protein or a transcription activator.

The three *whiB* genes: *whiB*1*, whiB*2 and *whiB*4 were PCR amplified with a oligonucleotide primers which had *Eco*RI site sequence in the 5' end and *Xho*I site sequence on their 3' end. The *whiB3* gene was amplified using *Bam*HI site in its 5' end because the *whiB* 3 gene has an internal *Eco*RI site. The PCR conditions were as described earlier. The PCR products were purified using a commercial purification kit and digested either with *Eco*RI and *Xho*I or *Bam*HI and *Xho*I restriction enzymes. The *E*. *coli* / *Saccharomyces cerevisiae* shuttle vector pEG202 was also digested appropriately, dephosphorylated and the PCR fragments were ligated into the vector by the method well know in the literature. The recombinants were selected on an ampicillin plate. The ampicillin positive clones were hybridized using the *whiB* genes as probes by the method well established in the literature. Plasmids were recovered from the clones that gave a positive signal by using a published method. To establish further that the correct gene has been cloned the plasmids were digested with appropriate restriction enzymes as described earlier and checked on an agarose gel for the correct size fragment.

Using a commercially available purification kit, large-scale plasmid preparation of the recombinant clones was made. Using a published protocol, 1µg of the recombinant plasmid was transformed into *Saccharomyces cerevisiae* EGY48 and EGY191 strains and selected for the complementation of histidine auxotrophy. The clones were patched on a histidine negative plate. The *Saccharomyces cerevisiae* EGY48 and EGY191 strains carrying recombinant *whiB* genes were co-transformed using various combinations of the plasmids:
(a) recombinant pEG202 + pSH18-34 + pJG4-5 (selected for histidine, uracil and tryptophan auxotrophy)
(b) pSH17-4 + pSH18-34+ pJG4-5 (selected for uracil and tryptophan auxotrophy)
(c) pRFHMI + pSHI 18-34 + pJG4-5 (selected for uracil and tryptophan auxotrophy)
(d) recombinant pEG202 + pJk101 (selected for histidine and uracil auxotrophy)
(e) pRFHM1 + pJK10 (selected for uraci auxotrophy)
(f) pJK101 (selected for uracil auxotrophy)
(g) recombinant pEG202+ pSH18-34 + pJG4-5 (selected for histidine, uracil and tryptophan auxotrophy).

Once the transformants complementing for the right auxotrophy were obtained, these clones were sub-cultured on a selective medium lacking those amino acids which was coded by the plasmids. Randomly selected six different clones were then streaked on the medium having following combinations:
(1) glucose⁺, ura⁻ BU salt⁺ +X-gal
(2) galactose/raffinose⁺, ura⁻ + BU salt⁺ + X-gal
(3) glucose⁺, ura⁻,his⁻,leu⁻
(4) galactose/raffinose⁺, ura⁻, his⁻, leu⁻
(5) galactose/raffinose⁺, ura⁻, his⁻, tryp⁻, leu⁻
(6) glucose⁺, ura⁻, his⁻, tryp⁻, leu⁻.
The plates containing X- gal were covered with aluminum foil to protect from light and were incubated at 30°C for 18-24hrs.

The *whiB*1*, whiB2* and *whiB*3 genes in the combination number (g) did not produce any blue color in an X-gal+, galactose/ raffinose+, ura-, BU+ plates nor did it grow on galactose/ raffinose+, ura-, his-, tryp- leu-plates. These combinations also did not induce leucine prototrophy. In contrast, the combination (d) repressed the *lacZ* induction when it was grown on an ura-, galactose/ raffinose+, BU+, X-gal+ plates, because they did not produce blue color however, the pJk101 alone (combination- f) produced blue color. These results confirmed that the *whiB*1*, whiB*2 and the *whiB*3 genes code for a DNA binding protein but not transcription activator.

The *whiB*4 gene is a weak transcription activator because unlike the recombinant *whiB*1, *whiB*2*,* and *whiB*3*,* the recombinant *whiB*4 in the combination number (g) produced light blue color in an X-gal+, galactose/ raffinose+, ura-, BU+ plates and also showed a weak activation of leucine prototrophy. This example establishes that the mycobacteria regulatory genes can be studied in an eukaryotic system -that is yeast and the *whiB* genes of the *Mycobacterium tuberculosis* H37Rv and *Mycobacterium bovis* BCG either code for DNA binding protein or a transcription activator.

### Example 5

To check whether *whiB* like genes are present in other species of mycobacteria or not, the following species of the mycobacteria were tested for the presence of *whiB* like genes: *Mycobacterium avium. Mycobacterium fortuitum, Mycobacterium gastri, Mycobacterium kansasii, Mycobacterium marinum, Mycobacterium microti, Mycobacterium phlei, Mycobacterium scrofulaceum, Mycobacterium smegmatis* and *Mycobacterium xenopi* using standard and published information. Except in *Mycobacterium fortuitum* which appear to have only *whiB*1homologue, the *whiB* genes homologues are present in all the species listed. The results suggest that one can use the *whiB* genes of the above species as well to develop a drug target.

### Summarising :

1. The *whiB* genes of the *Mycobacterium tuberculosis* H37Rv and *Mycobacterium bovis* BCG are regulatory genes.
2. The disruption of *whiB* genes is either lethal or deleterious for the survival of mycobacteria.
3. The *whiB* genes are used as drug target to search for anti-tuberculosis drugs.
4. The yeast two-hybrid system can be used to study the mycobacterial regulatory genes and then to study to what genes are controlled by these regulatory genes.
5. This is the first report where yeast two-hybrid system has been used to investigate the nature of a protein of *Mycobacterium tuberculosis,* which so far, had only predicted function.
6. A method is described wherein all genetically engineered but truncated regulatory genes can also be studied. These studies are done to delineate the function of different regions of the regulatory genes.
7. The invention also provides means to study protein-protein interactions of mycobacterial genes in a heterologous host that is not infectious thus does not pose a health hazard or demand special laboratory set-up.
8. Also described herein is a reporter gene based assay that uses a well-characterized gene *lacZ.* The *lacZ* gene codes for the β-galactosidase. The assay of β -galactosidase enzyme is simple, thus the method is also HTS compatible.

## Claims

1. A method for making a recombinant *Saccharomyces-cerevisiae,* said method comprising the steps of:
a) amplifying the whiB-like genes of *Mycobacteria* by the polymerase chain reaction (PCR), wherein the whiB-like genes are selected from the group consisting of whiB1 (SEQ ID NO:1), whiB2 (SEQ ID NO:3), whiB3 (SEQ ID NO:5), and whiB4 (SEQ ID NO:7) and the Mycobacteria are selected from the group consisting of *Mycobacterium tuberculosis, Mycobacterium bovis, Mycobacterium avium, Mycobacterium fortuitum, Mycobacterium gastri, Mycobacterium kansasii, Mycobacterium leprae, Mycobacterium marinum, Mycobacterium microti, Mycobacterium phlei, Mycobacterium scrofulaceum, Mycobacterium smegmatis and Mycobacterium xenopi,* and wherein the *whiB* genes are amplified with primers carrying either EcoRl or BamHI restriction enzyme sites at the 5' end and a Xhol restriction enzyme site at the 3' end;
b) digesting the fragment as obtained in step a) with suitable restriction enzymes and then cloning the fragment into an *E. coli*/*Saccharomyces cerevisiae* shuttle vector pEG202 to produce the cloned gene as a *LexA* fusion protein and transforming the clone into E.coli; and
c) amplifying the clone and then transforming the recombinant *whiB1, whiB2, whiB3* and *whiB4*/pEG202 into a strain of *Saccharomyces cerevisiae* which has *LexA* operators.

2. The method as claimed in claim 1, wherein the *Saccharomyces cerevisiae* is a strain selected from EGY48 or EGY191.

3. The method as claimed in claim 1, wherein the whiB1 gene is amplified using primers F 5' acccgttaccagccaagaag 3' (SEQ ID NO:9) and R 5' gggacggttgatgctgtag 3' (SEQ ID NO:10).

4. The method as claimed in claim 1, wherein the whiB2 gene is amplified using primers F 5' ggccgggtcagatgatc 3' (SEQ ID NO:11) and R 5' accgcatctgagtttgg 3' (SEQ lD NO:12).

5. The method as claimed in claim 1, wherein the whiB3 gene is amplified using primers F 5' atgccacagccggagcagctac 3' (SEQ ID NO:13) and R 5' ttaagctgtgcggcggatgcc 3' (SEQ lD NO:14).

6. The method as claimed in claim 1, wherein the whiB4 gene is amplified using primers F 5' ctatccggcggtgccggtgcg 3' (SEQ ID NO:15) and R 5' gtggtacgcagcgtagacgcg 3' (SEQ ID NO:16).

7. The method as claimed in claim 1, wherein the restriction enzymes used in step b) are selected from a group consisting of EcoRl, Xmal BamHl, Sail, Ncol, Notl, Xhol, Sall and Pstl.

## Patentansprüche

1. Verfahren zur Herstellung eines rekombinanten *Saccaromyces cerevisiae,* wobei das Verfahren die folgenden Schritte umfasst:
a) Vervielfältigen der whiB-artigen Gene von *Mycobacteria* durch die Polymerase-Kettenreaktion (polymerase chain reaction, PCR), wobei die whiB-artigen Gene aus der Gruppe ausgewählt sind, die aus whiB1 (SEQ ID NR: 1), whiB2 (SEQ ID NR: 3), whiB3 (SEC ID NR: 5) und whiB4 (SEQ ID NR: 7) besteht, und die Mycobacteria aus der Gruppe ausgewählt sind, die aus *Mycobacterium tuberculosis, Mycobacterium bovis, Mycobacterium avium, Mycobacterium fortuitum, Mycobacterium gastri, Mycobacterium kansasii, Mycobacterium leprae, Mycobacterium marinum, Mycobacterium microti, Mycobacterium phlei, Mycobacterium scrofulaceum, Mycobacterium smegmatis und Mycobacterium xenopi* besteht, und wobei die whiB Gene mit Primem verstärkt werden, die entweder EcoRI- oder BamHI- Restriktionsenzymstellen an ihrem 5'-Ende und eine XhoI-Restriktionsenzymstelle an dem 3'-Ende tragen;
b) Verdauen des wie in Schritt a) erhaltenen Fragments mit geeigneten Restriktionsenzymen und anschließendes Klonieren des Fragments in einen *E.coli*/ *Saccharomyces cerevisiae*-Shuttlevektor pEG202, um das klonierte Gen als ein LexA-Fusionsprotein herzustellen und den Klon in E.coli zu transformieren; und
c) Vervielfältigen des Klons und anschließendes Transformieren der rekombinanten whiB1 , whiB2, whiB3 und whiB4/pEG202 in einen Stamm *Saccharomyces cerevisiae,* der *LexA*-Operatoren aufweist.

2. Verfahren nach Anspruch 1, wobei das *Saccharomyces cerevisiae* ein aus EGY48 oder EGY191 ausgewählter Stamm ist.

3. Verfahren nach Anspruch 1, wobei das whiB1-Gen durch Verwendung von Primem F 5' acccgttaccagccaagaag 3' (SEQ ID NR: 9) und R 5' gggacggttgatgctgtag 3' (SEQ ID NR: 10) vervielfältigt wird.

4. Verfahren nach Anspruch 1, wobei das whiB2-Gen durch Verwendung von Primem F 5' ggccgggtcagatgatc 3' (SEQ ID NR: 11) und R 5' accgcatctgagtttgg 3' (SEQ ID NR: 12) vervielfältigt wird.

5. Verfahren nach Anspruch 1, wobei das whiB3-Gen durch Verwendung von Primem F 5' atgccacagccggagcagctac 3' (SEC ID NR: 13) und R 5' ttaagctgtgcggcggatgcc 3' (SEQ ID NR: 14) vervielfältigt wird.

6. Verfahren nach Anspruch 1, wobei das whiB4-Gen durch Verwendung von Primem F 5' ctatccggcggtgccggtgcg 3' (SEQ ID NR: 15) und R 5' gtggtacgcagcgtagacgcg 3' (SEQ ID NR: 16) vervielfältigt wird.

7. Verfahren nach Anspruch 1, wobei die Restriktionsenzyme, die in Schritt b) verwendet werden, aus der Gruppe ausgewählt werden, die aus EcoRI, XmaI BamHI, SaiI, NcoI, NotI, XhoI, SaII und PstI besteht.

## Revendications

1. Procédé de production d'une *Saccharomyces cerevisiae* recombinante, ladite méthode comprenant les étapes :
a) d'amplification des gènes apparentés à whiB de *Mycobacteria* par amplification par polymérisation en chaîne (PCR), dans lequel les gènes apparentes à whiB sont sélectionnés dans le groupe constitué de whiB1 (SEQ ID NO:1), whiB2 (SEQ ID NO:3), whiB3 (SEQ ID NO:5), et de whiB4 (SEQ ID NO:7) et les mycobactéries sont sélectionnées dans le groupe constitué de *Mycobacterium tuberculosis, Mycobacterium bovis, Mycobacterium avium, Mycobacterium fortuitum, Mycobacterium gastri, Mycobacterium kansasii, Mycobacterium leprae, Mycobacterium marinum, Mycobacterium microti, Mycobacterium phlei, Mycobacterium scrofulaceum, Mycobacterium smegmatis* et *Mycobacterium xenopi,* et dans lequel les gènes apparentes à whiB sont amplifiés avec des amorces portant soit des sites d'enzyme de restriction EcoRI ou BamHI à l'extrémité 5' et un site d'enzyme de restriction XhoI à l'extrémité 3' ;
b) de digestion du fragment tel qu'obtenu lors de l'étape a) avec des enzymes de restriction adaptées, puis de clonage du fragment dans un vecteur navette *E. coli*/*Saccharomyces cerevisiae* pEG202 pour produire le gène cloné comme une protéine de fusion LexA, et de transformation du clone dans E.coli ; et
c) d'amplification du clone puis de transformation du recombinant *whiB1, whiB2, whiB3* et *whiB4*/*pEG202* dans une souche de *Saccharomyces cerevisiae* possédant des opérateurs LexA.

2. Procédé selon la revendication 1, dans lequel la *Saccharomyces cerevisiae* est une souche choisie entre EGY48 ou EGY191.

3. Procédé selon la revendication 1, dans lequel le gène whiB1 est amplifié par l'utilisation des amorces F 5' acccgttaccagccaagaag 3' (SEQ ID NO:9) et R 5' gggacggttgatgctgtag 3' (SEQ ID NO:10).

4. Procédé selon la revendication 1, dans lequel le gène whiB2 est amplifié par l'utilisation des amorces F 5' ggccgggtcagatgatc 3' (SEQ ID NO:11) et R 5' accgcatctgagtttgg 3' (SEQ ID NO:12).

5. Procédé selon la revendication 1, dans lequel le gène whiB3 est amplifié par l'utilisation des amorces F 5' atgccacagccggagcagctac 3' (SEQ ID NO:13) et R 5' ttaagctgtgcggcggatgcc 3' (SEQ ID NO:14).

6. Procédé selon la revendication 1, dans lequel le gène whiB4 est amplifié par l'utilisation des amorces F 5' ctatccggcggtgccggtgcg 3' (SEQ ID NO:15) et R 5' gtggtacgcagcgtagacgcg 3' (SEQ ID NO:16).

7. Procédé selon la revendication 1, dans lequel les enzymes de restriction utilisées lors de l'étape b) sont sélectionnées dans un groupe constitué de EcoRI, Xmal BamHI, SaiI, NcoI, NotI, XhoI, SalI and PstI.
